# EUROPEAN PATENT SPECIFICATION

(11) **EP 4 029 548 B1**
(45) Date of publication and mention of the grant of the patent: **06.08.2025**
(21) Application number: 21152383.2
(22) Date of filing: 19.01.2021
(51) Int. Cl.: A61M 5/315, A61M 5/28, A61M 5/31

(54) **STOPPER ASSEMBLY FOR PREFILLED INJECTION DEVICE**
STOPFENANORDNUNG FÜR VORGEFÜLLTE INJEKTIONSVORRICHTUNG
ENSEMBLE D'ARRÊT D'UN DISPOSITIF D'INJECTION PRÉREMPLI

(43) Date of publication of application: 20.07.2022
(73) Proprietor: Becton Dickinson Holdings Pte. Ltd., Singapore 639461 (SG)
(72) Inventor: NALAWADE, Praveen, Karnataka, 560054 BANGALURU (IN); SAPINENI, Raghuveera, 560054 BANGALORE (IN)
(74) Representative: Regimbeau

(56) References cited:
- EP-A2- 0 111 724
- EP-A2- 0 743 072
- WO-A2-2015/054282
- US-A1- 2018 028 758

## Description

### TECHNICAL FIELD

The present disclosure relates generally to a stopper assembly and an injection device comprising the stopper assembly.

### BACKGROUND

Injection devices, such as syringes, are medical delivery devices configured to administer a medicinal fluid (e.g., pharmaceutical, medicament) to a patient. Syringes may be provided in a prefilled form, wherein medicinal fluid is disposed within the syringe at, for example, the time of manufacture of the syringe, or in an empty - or prefillable - form, wherein medicinal fluid is filled from a vial or other source by an end user close in time to injecting the medicinal fluid with the syringe.

Syringes commonly comprise a stopper displaceable by a plunger within a syringe barrel in order to administer the medicinal fluid to a patient. With respect to prefilled syringes, the storage time of the syringe, or the time between filling the syringe with medicinal fluid and administering the medicinal fluid to the patient, can vary widely. As the storage time increases, the interaction between the medicinal fluid and the stopper may affect the potency of the stored medicinal fluid. Maintaining proper drug potency for the duration of storage is important to user safety and efficacy of treatment with the medicinal fluid. Examples of syringes including stoppers and stopper assemblies are given in EP0743072, WO2015/054282, US2018/028758, and EP0111724.

One solution to prevent contact between the medicinal fluid and the stopper is provided in US Patent No. 6,090,081. The sealing stopper of the syringe of US Patent No. 6,090,081 is provided with a resin film laminated along an exterior surface of the sealing stopper such that the resin film is in contact with the medicinal fluid and in contact with an inner surface of the barrel along which the sealing stopper slides. This solution requires the material of the resin film to be tested for compatibility with the material of the sealing stopper in order to assure proper lamination and for compatibility with the medicinal fluid. As a result, additional regulatory approval may be required. Further, an actuation force that must be applied by the user to the plunger in order to displace the sealing stopper within the barrel may vary compared to uncoated sealing stoppers. As a result, the injection rate may be unexpectedly modified for the user. Therefore, there is a need for an injection device that overcomes the foregoing setbacks.

### BRIEF SUMMARY

An objective of the present disclosure is to provide a stopper assembly that fluidly isolates a stopper from a medicinal fluid such that the medicinal fluid may be stored in the injection device without a change in the potency of the medicinal fluid.

Another objective of the present disclosure is to provide the aforementioned stopper assembly that does not affect the actuating force applied to a plunger rod of an injection device including the stopper assembly such that the actuating force may be the same as the actuating force applied for an injection device comprising a stopper without a sealing element as described herein.

To this end, the present disclosure describes a stopper assembly configured to be disposed in a barrel of an injection device containing a medicinal fluid. The stopper assembly comprises a stopper and a sealing element. The stopper has a laterally outermost surface configured to be directly engaged with an inner surface of the barrel. The sealing element is coupled to and axially distal relative to the stopper in the barrel. The sealing element has a laterally outermost surface configured to be directly engaged with the inner surface of the barrel and to sealingly isolate the stopper from the medicinal fluid in the barrel.

Certain preferred but non-limiting features of the stopper assembly described above are the following, taken individually or in combination:
the sealing element comprises a polymeric material chemically inert to the medicinal fluid to be disposed within the barrel;
the sealing element comprises a distal portion and a proximal portion, the distal portion having a maximum lateral dimension greater than a maximum lateral dimension of the proximal portion;
the stopper has a distal surface and a proximal surface;
the stopper comprises a cavity extending into the stopper from the distal surface thereof;
the proximal portion of the sealing element is disposed in the cavity of the stopper;
the cavity comprises at least one laterally-extending groove and the proximal portion of the sealing element comprises at least one laterally-extending projection, the at least one laterally-extending projections received in and engaged with the laterally-extending grooves to couple the stopper and the sealing element;
the laterally outermost surface of the sealing element has a surface roughness in a range from about 0.012 µm to about 0.10 µm, inclusive; and/or
the distal portion of the sealing element is hemispherical in shape, conical in shape, or disc-shaped.

According to the invention, there is provided an injection device as defined in claim 1 and in the corresponding dependent claims. The invention proposes an injection device comprises a barrel, a plunger rod, and the stopper assembly described above disposed in the barrel. The barrel extends axially between a distal end and a proximal end. The barrel is configured to contain a medicinal fluid. The plunger rod has a distal end and a proximal end. The distal end of the plunger rod is disposed in the barrel.

Certain preferred but non-limiting features of the injection device described above are the following, taken individually or in combination:
the stopper is sealingly enclosed between the sealing element and the plunger;
a maximum lateral dimension of the inner surface of the barrel is less than the maximum lateral dimension of the distal portion of the sealing element such that the distal portion of the sealing element is laterally compressed within the barrel in the storage position;
a maximum lateral dimension of the stopper is greater than the maximum lateral dimension of the inner surface of the barrel such that the stopper is laterally compressed within the barrel;
the lateral compression of the stopper applies a compressive retaining force on the proximal portion of the sealing element disposed in the cavity of the stopper;
the stopper assembly is configured to be axially movable from a storage position to a use position within the barrel;
the barrel comprises a retaining ring proximate to the proximal end thereof; and
the stopper assembly is located axially distal relative to the retaining ring in the storage position;
the barrel further comprises another retaining ring located proximate to the proximal end of the barrel, the retaining ring axially spaced apart from and located proximal relative to the another retaining ring, the retaining rings defining a minimum lateral dimension of the inner surface of the barrel;
each of the sealing element, the stopper, and a flange of the plunger rod is located between the retaining rings in the storage position such that the stopper is axially compressed between the sealing element and the flange of the plunger; and/or
the maximum lateral dimension of the inner surface of the barrel axially distal relative to the first and second retaining rings is greater than the maximum lateral dimension of the proximal portion of the sealing element such that the laterally outermost surface of the sealing element is disengaged from the inner surface of the barrel in the use position.

### BRIEF DESCRIPTION OF THE DRAWINGS

The illustrations presented herein are not meant to be actual views of any particular component, device, or system, but are merely idealized representations which are employed to describe embodiments of the present invention. Other features, goals, and advantages of the present invention will appear more clearly upon reading of the detailed description that follows and with references to drawings provided by way of non-limiting examples wherein:
FIG. 1A is a cross-sectional view of an injection device comprising a stopper assembly;
FIG. 1B is an enlarged view of the stopper assembly within the injection device of FIG. 1A;
FIGS. 2A and 2B are a perspective view and cross-sectional view, respectively, of a sealing element of the stopper assembly;
FIGS. 3A and 3B are a perspective view and cross-sectional view, respectively, of a stopper of the stopper assembly;
FIG. 4A is a cross-sectional view of another injection device comprising a stopper assembly;
FIG. 4B is an enlarged view of the stopper assembly within the injection device of FIG. 4A;
FIGS. 5A and 5B are a perspective view and cross-sectional view, respectively, of another sealing element of the stopper assembly; and
FIG. 6 is a cross-sectional view of a barrel of the injection device of FIG. 4A.

### DETAILED DESCRIPTION

As used herein, the term "proximal" refers to a location, such as a proximal end, that is nearer to a point of reference such as a point of contact of a user applying a force to a plunger rod of an injection device as described herein. As used herein, the term "distal" refers to a location, such as a distal end, that is farther from a point of reference such as a point of contact of the user applying a force to the plunger of the injection device as described herein. Thus, the terms "proximal" and "distal" refer to, for example, directions nearer to and farther from, respectively a user administering a medicinal fluid to a patient.

As used herein, the terms "axial," "axially," "longitudinal," and "longitudinally" generally mean and refer to a direction along or parallel to a longitudinal axis of an element(s) of the injection device described herein.

As used herein, the terms "radial," "radially," "lateral," and "laterally" generally mean and refer to a direction perpendicular to the central, longitudinal axis of the element(s) of the injection device described herein.

As used herein, the term "and/or" includes any and all combinations of one or more of the associated listed items.

As used herein, the term "configured" refers to a size, shape, material composition, material distribution, orientation, and arrangement of one or more of at least one structure and at least one apparatus facilitating operation of one or more of the structure and the apparatus in a pre-determined way.

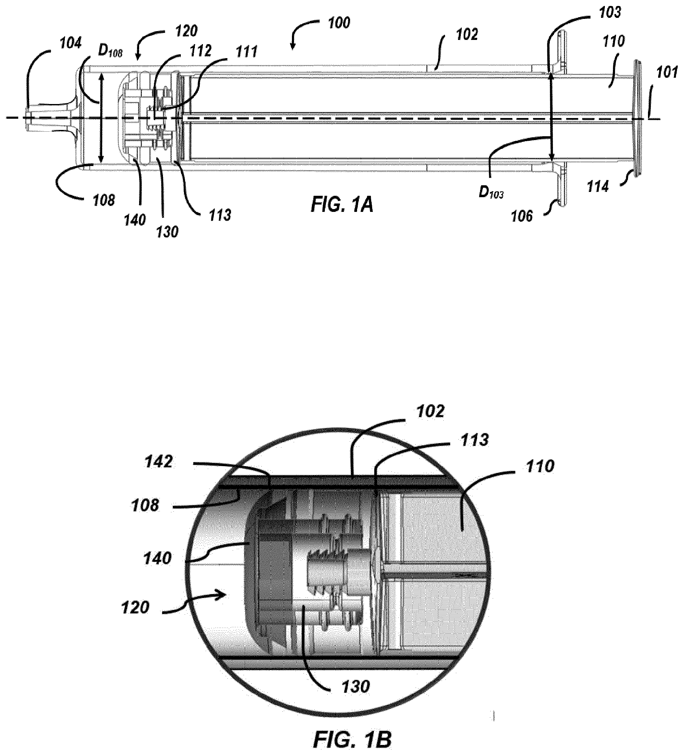

FIG. 1A illustrates a cross-sectional view of an injection device 100, which may also be referred to herein as a syringe. The injection device 100 may comprise a barrel 102 extending axially along a longitudinal axis 101 between a distal end 104 and a proximal end 106. The barrel 102 is configured to contain a medicinal fluid therein. A cannula (e.g., needle) may be coupled to the distal end 104 for injecting the medicinal fluid to a patient. The needle may be stacked on the distal end 104 or may be coupled, such as by threading, to the distal end 104 via an adaptor, which may be snap-fitted or glued to the distal end 104.

The barrel 102 comprises an inner surface 108 defining a hollow space in which a stopper assembly according to embodiments of the present disclosure may be disposed. The inner surface 108 defines a maximum lateral dimension D₁₀₈ of the barrel 102. When the barrel 102 is circular in cross-sectional shape, the maximum lateral dimension D₁₀₈ may be a maximum diameter of the inner surface 108 of the barrel 102.

The barrel 102 may comprise a retaining ring 103 proximate to the proximal end 106 thereof. The retaining ring 103 defines a portion of the inner surface 108 of the barrel 102. The retaining ring 103 further defines a minimum lateral dimension D₁₀₃ of the barrel 102. When the barrel 102 is circular in cross-sectional shape, the minimum lateral dimension D₁₀₃ may be a minimum diameter of the inner surface 108 of the barrel 102. As explained in further detail below, the retaining ring 103 may be sized and configured to inhibit passage of a stopper assembly 120 distally therebeyond unless a force greater than or equal to a predetermined actuating force is applied to a plunger rod 110.

The barrel 102 may be formed of a polymeric material (e.g., plastic) or glass.

A stopper assembly 120 may be disposed in the barrel 102. FIG. 1B provides an enlarged cross-sectional view of the stopper assembly 120 within the barrel 102. The stopper assembly 120 may comprise a stopper 130 and a sealing element 140.

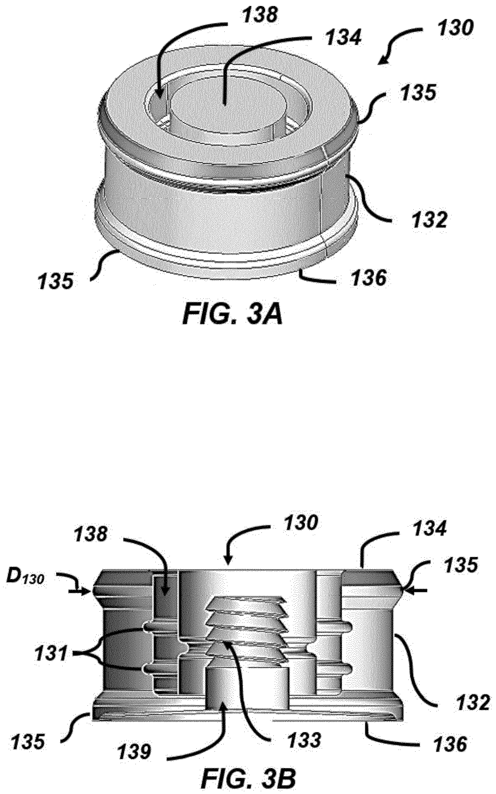

The stopper 130 is shown in isolation from the injection device 100 in FIGS. 3A and 3B. The stopper 130 has a laterally outer surface 132. The laterally outer surface 132 defines a lateral dimension D₁₃₀ of the stopper 130. The stopper 130 also has a distal surface 134 and a proximal surface 136. When the stopper 130 is circular in cross-sectional shape, the lateral dimension D₁₃₀ of the stopper 130 is the diameter of the stopper 130.

The laterally outer surface 132 may comprise a plurality (e.g., two, three, or more) of rings 135. As illustrated in FIGS 3B, in some embodiments, the laterally outer surface 132 comprises a first ring 135 proximate to the distal surface 134 and a second ring 135 proximate to the proximal end 136. The rings 135 define laterally outermost surfaces of the stopper 130 such that a remainder of the laterally outer surface 132 of the stopper 130 may be laterally recessed relative to rings 135. Alternatively, the stopper 130 may have a constant lateral dimension D₁₃₀.

The stopper 130 may comprise a cavity 138 extending into the stopper 130 from the distal surface 134. The cavity 138 may comprise at least one laterally-extending groove 131. The laterally-extending groove(s) 131 extends at least partially or entirely about a periphery of a surface of the stopper 130 defining the cavity 138.

The stopper 130 may comprise another cavity 139 extending into the stopper 130 from the proximal surface 136. The another cavity 139 may comprise a threaded surface 133 on a surface of the stopper 130 defining the another cavity 139.

The stopper 130 may be formed of a polymeric material. By way of non-limiting example, the stopper 130 may be formed of an elastomeric material. The elastomeric material may be selected from the group consisting of natural rubber, synthetic rubber, thermoplastic elastomers, or combinations thereof.

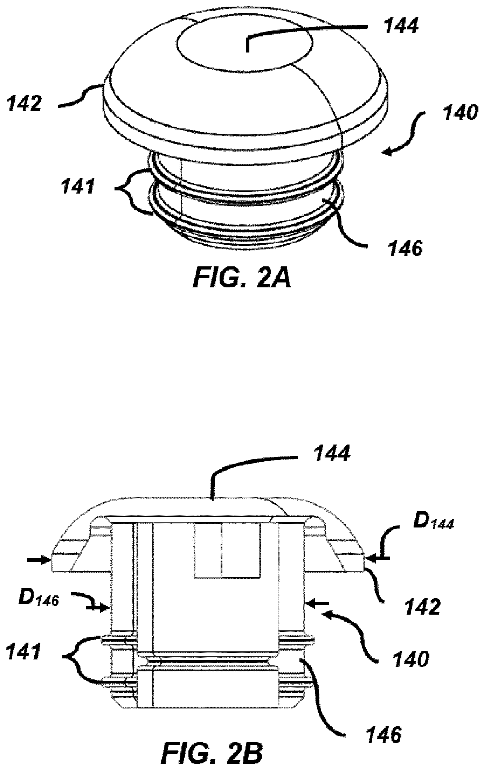

The sealing element 140 is shown in isolation from the injection device 100 in FIGS. 2A and 2B. The sealing element 140 may comprise a distal portion 144 and a proximal portion 146. The sealing element 140 has a laterally outermost surface 142. The laterally outermost surface 142 that may be configured to be directly engaged (e.g., in contact with) with the inner surface 108 of the barrel 102.

The distal portion 144 has a maximum lateral dimension D₁₄₄ greater than a maximum lateral dimension D₁₄₆ of the proximal portion 146. The distal portion 144 may have a hemispherical-shape as shown in the figures. However, the shape of the distal portion 144 is not so limited and may have another shape, such as a disc shape or a conical shape.

The distal portion 144 may comprise the laterally outermost surface 142 of the sealing element 140. Accordingly, the maximum lateral dimension D₁₄₄ of the distal portion 144 may be a maximum lateral dimension of the sealing element 140.

When the distal portion 144 and/or proximal portion 146 of the sealing element 140 are circular in cross-sectional shape, the maximum lateral dimension D₁₄₆ and maximum lateral dimension D₁₄₄ may be the maximum diameter of the distal portion 144 and proximal portion 146 respectively.

The proximal portion 146 may comprise at least one laterally-extending projection 141. The laterally-extending projection(s) 141 may extend at least partially or entirely about a periphery of the proximal portion 146.

The sealing element 140 may be formed of a polymeric material. More particularly, the polymeric material of the sealing element 140 is selected to be chemically inert to the medicinal fluid to be disposed within the barrel 102. The polymeric material may be a rigid or semi-rigid polymer material such that axial deformation of the sealing element 140 is sufficiently minimal as to avoid blood reflux as described in further detail below.

When the barrel 102 comprises a polymeric material, the barrel 102 and the sealing element 140 may be formed from the same polymeric material. The polymeric material may be selected from the group consisting of polyolefin, polyamide, polyester, polypropylene, polystyrene, polyurethane, polycarbonate, acrylonitrile butadiene styrene, fluoropolymer, ionomer, polyacrylate, or any combination thereof.

The laterally outermost surface 142 of the sealing element 140 may be formed to have a pre-determined surface roughness. The surface roughness may be modified or otherwise selected based on the desired actuating force.

The sealing element 140 may be formed in an injection molding process. A surface of a mold, which is used to form the laterally outermost surface 142, may be configured to impart the desired surface roughness.

In some embodiments, the laterally outermost surface 142 may have a surface roughness in a range extending from 0.012 µm to about 0.10 µm, inclusive, and, more preferably, in a range extending from 0.05 µm to about 0.10 µm, inclusive. Accordingly, the laterally outermost surface 142 may have one of a super high glossy finish (i.e., grade A-1 according to the Society of Plastics Industry (SPI) standard, a high glossy finish (i.e., a grade A-2 according to the SPI standard), a normal glossy finish (i.e., a grade A-3 according to the SPI standard), or a fine semi-glossy finish (i.e., a grade B-1 according to the SPI standard).

When the stopper 130 and the sealing element 140 are assembled together to form the stopper assembly 120, a portion of the sealing element 140 is disposed within the cavity 138 of the stopper 130. In particular, the proximal portion 146 of the sealing element 140 may be disposed in the cavity 138 of the stopper 130.

When the proximal portion 146 of the sealing element 140 is disposed in the cavity 138 of the stopper 130, the laterally-extending projection(s) 141 are disposed in the laterally-extending groove(s) 131. Disposing the laterally-extending projection(s) 141 of the sealing element 140 in the laterally-extending groove(s) 131 of the stopper 130 provides a coupling mechanism between the sealing element 140 and the stopper 130. Thus, when the stopper assembly 120 is displaced within the barrel 102 in operation, the sealing element 140 and the stopper 130 remain coupled together.

When the stopper assembly 120 is disposed in the barrel 102, the laterally outer surface 132 of the stopper 130 is directly engaged with the inner surface 108 of the barrel 102.

The lateral dimension D₁₃₀ of the stopper 130 is greater than the maximum lateral dimension D₁₀₈ and the minimum lateral dimension D₁₀₃. Due to the dimensions of the stopper 130 relative to the dimensions of the barrel 102, the stopper 130 is laterally compressed within the barrel 102 when the stopper assembly 120 is disposed therein. The lateral compression of the stopper 130 exerts a compressive force on the proximal portion 146 of the sealing element 140 disposed in the cavity 138 of the stopper 130. In addition to or as an alternative to providing the laterally-extending grooves 131 and projections 141, the laterally compressive force of the stopper 130 on the sealing element 140 provides a coupling mechanism between the sealing element 140 and the stopper 130. Thus, when the stopper assembly 120 is displaced within the barrel 102 in operation, the sealing element 140 and the stopper 130 remain coupled together.

When the stopper assembly 120 is disposed in the barrel 102, the sealing element 140 is located axially distal relative to the stopper 130. By disposing the sealing element 140 axially distal relative to the stopper 130, the sealing element 140 is in contact with the medicinal fluid also disposed within the barrel 102. Thus, the sealing element 140 sealingly isolates the stopper 130 from the medicinal fluid. As a result of the isolation of the stopper 130 from the medicinal fluid, the potency of the medicinal fluid remains unchanged when the medicinal fluid is stored within the barrel 102 over time.

When the stopper assembly 120 is disposed in the barrel 102, the laterally outermost surface 142 of the sealing element 140 may be directly engaged with the inner surface 108 of the barrel 102. Further, during storage/transport of a prefilled syringe, the proximal portion 146 sealing element 140 may initially abut against a distal part of the retaining ring 103.

The maximum lateral dimension D₁₀₈ of the inner surface 108 of the barrel 102 may be less than the maximum lateral dimension D₁₄₄ of the distal portion 144 of the sealing element 140. Accordingly, when the stopper assembly 120 is disposed in the barrel 102, the distal portion 144 of the sealing element 140 is laterally compressed. Lateral compression of the sealing element 140 forms a fluid tight interface between the sealing element 140 and the inner surface 108 of the barrel 102. Thus, in addition to the axial location of the sealing element 140 relative to the stopper 130, the dimension of the sealing element 140 sealingly isolates the stopper 130 from the medicinal fluid. In other words, the medicinal fluid disposed in the barrel 102 is in contact with the sealing element 140 and cannot contact the stopper 130 due to the fluid tight interface between the sealing element 140 and the inner surface 108 of the barrel 102. As a result of the isolation of the stopper 130 from the medicinal fluid, the potency of the medicinal fluid remains unchanged when the medicinal fluid is stored within the barrel 102 over time.

The stopper assembly 120 is initially disposed in the storage position within the barrel 102. In the storage position, the stopper assembly 120 is located axially distal relative to the retaining ring 103. As previously described herein, the maximum lateral dimension D₁₄₄ of the sealing element 140 and the lateral dimension D₁₃₀ of the stopper 130 are greater than the minimum lateral dimension D₁₀₃ of the retaining ring 103. The sealing element 140 and the stopper 130 may be compressed to allow passage of the stopper assembly 120 between and distally beyond the retaining ring 103.

The injection device 100 may further comprise a plunger rod 110. The plunger rod 110 has a distal end 112 and a proximal end 114. The distal end 112 of the plunger rod 110 may be disposed in the barrel 102.

The distal end 112 of the plunger rod 110 may be coupled to the stopper assembly 120. The distal end 112 of the plunger rod 110 may comprise a threaded surface 111. As previously described, the another cavity 139 of the stopper 130 may comprise the threaded surface 133, which is configured to couple the plunger rod 110 to the stopper 130.

The plunger rod 110 may comprise a flange 113. When the plunger rod 110 is coupled to the stopper 130, the stopper 130 may be disposed between the flange 113 of the plunger rod 110 and the sealing element 140.

In operation, the stopper assembly 120 is axially displaced in a distal direction from a storage position to a use position. FIG. 1A illustrates the stopper assembly 120 in the use position. As previously described, the stopper assembly 120 is located axially distal relative to the retaining ring 103 in the storage position. To axially displace the stopper assembly 120 and to administer the medicinal fluid to a patient, a force is applied by a user on the proximal end 114 of the plunger rod 110. The applied force must be greater than or equal to a predetermined actuating force. The maximum lateral dimension D₁₄₄ of the sealing element 140, the maximum lateral dimension D₁₃₀ of the stopper 130, the surface roughness of the laterally outermost surface 142 of the sealing element 140, and/or the material composition of the sealing element 140 and/or stopper 130 may be selectively tailored to obtain a desired, predetermined actuating force. While the stopper assembly 120 is axially displaced in a distal direction within the barrel 102, the stopper 130 remains sealingly isolated from the medicinal fluid by the sealing element 140.

The plunger rod 110 may continue to be axially displaced (e.g., distally displaced) until the sealing element 140 abuts against a distal end of the inner surface 108 of the barrel 102. For injection devices lacking the sealing element 140, a syringe-induced blood reflux may occur. Syringe-induced blood reflux refers to the drawing back of blood into the injection device caused by the generation of a vacuum within the barrel from the compression and subsequent rebound of a rubber stopper against the distal end of the inner surface of the barrel. The sealing element 140, which is made of a rigid or semi-rigid polymeric material as described herein, does not axially compress and otherwise prevents such compression and subsequent rebound of the stopper 130 because the stopper 130 does not abut against the distal end of the inner surface 108 of the barrel 102. Thus, a further advantage of the stopper assembly 120 is the avoidance of blood reflux. It is to be understood that the stopper assembly 120 of the invention may also be used in reconstitution device.

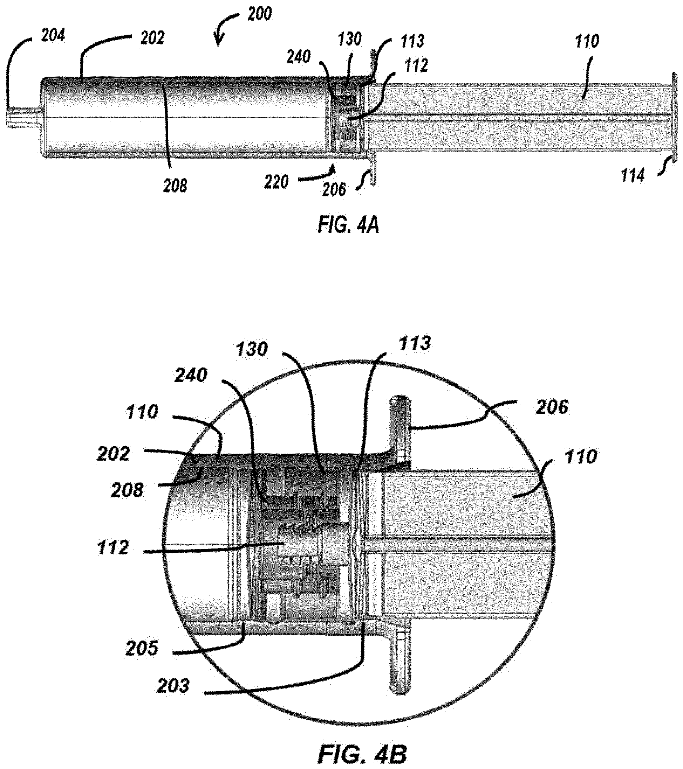

FIG. 4A illustrates a cross-sectional view of an injection device 200. The injection device 200 may comprise a barrel 202 extending axially along a longitudinal axis 201 between a distal end 204 and a proximal end 206. The barrel 202 is configured to contain a medicinal fluid therein. A cannula (e.g., needle) may be coupled to the distal end 204 for administering the medicinal fluid to a patient.

The barrel 202 comprises an inner surface 208 defining a hollow space in which a stopper assembly according to embodiments of the present disclosure may be disposed. The inner surface 208 defines a maximum lateral dimension D₂₀₈ of the barrel 202. When the barrel 202 is circular in cross-sectional shape, the maximum lateral dimension D₂₀₈ may be a maximum diameter of the inner surface 208 of the barrel 202.

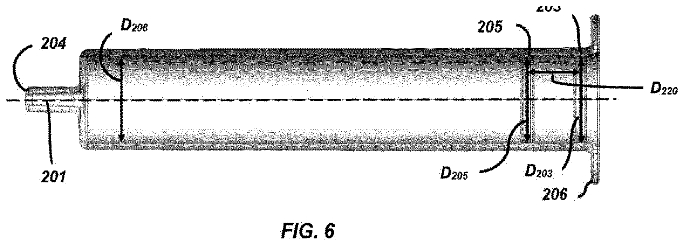

Like the barrel 102, the barrel 202 may comprise at least one retaining ring. As best illustrated in FIG. 6, the barrel 202 comprises a retaining ring 203 and another retaining ring 205 located proximate to the proximal end 206 thereof. The retaining ring 203 is located proximal relative to the retaining ring 205. The retaining rings 203, 205 are axially spaced apart by an axial distance D₂₂₀.

The retaining rings 203, 205 define a portion of the inner surface 208 of the barrel 202. The retaining rings 203, 205 further defines a minimum lateral dimension D₂₀₃, D₂₀₅ of the barrel 202. When the barrel 202 is circular in cross-sectional shape, the minimum lateral dimension D₂₀₃, D₂₀₅ may be minimum diameters of the inner surface 208 of the barrel 202.

As explained in further detail below, the retaining rings 203, 205 may be sized and configured to inhibit passage of a stopper assembly 220 distally therebeyond unless a force greater than or equal to a predetermined actuating force is applied to a plunger rod 110.

The barrel 202 may be formed from the same materials previously described with reference to barrel 102.

A stopper assembly 220 may be disposed in the barrel 202. FIG. 4B provides an enlarged cross-sectional view of the stopper assembly 220 within the barrel 202. The stopper assembly 220 may comprise the stopper 130 previously described with reference to FIGS. 3A and 3B and a sealing element 240.

While the stopper assembly 220 is described primarily with reference to its disposal within the barrel 202, the stopper assembly 220 may also be used in combination with barrel 102.

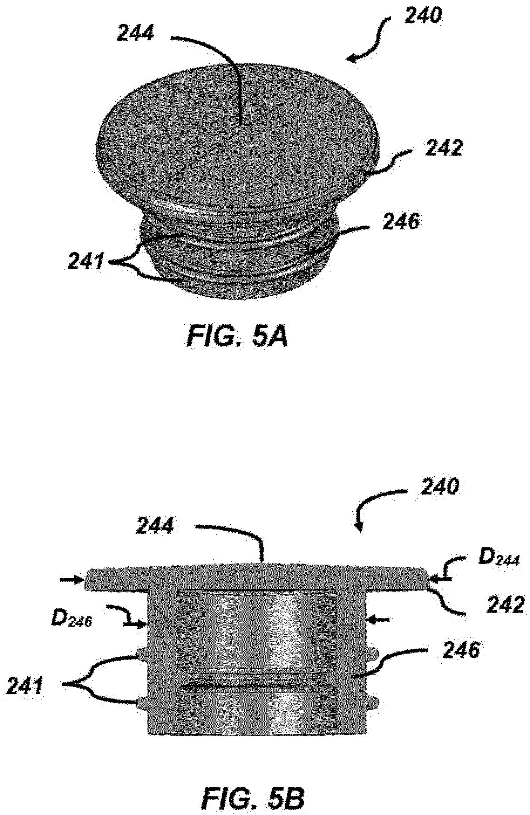

The sealing element 240 is shown in isolation from the injection device 200 in FIGS. 5A and 5B. The sealing element 240 may comprise a distal portion 244 and a proximal portion 246. The sealing element 240 has a laterally outermost surface 242.

The distal portion 244 has a maximum lateral dimension D₂₄₄ greater than a maximum lateral dimension D₂₄₆ of the proximal portion 246. The distal portion 244 may be disc-shaped. Accordingly, a distal surface of the distal portion 244 may be planar. Alternatively, the distal surface of the distal portion 244 may be curved (e.g., hemispherical) or conical.

When the distal portion 244 and/or proximal portion 246 of the sealing element 240 are circular in cross-sectional shape, the maximum lateral dimension D₂₄₆ and maximum lateral dimension D₂₄₄ may be the maximum diameter of the distal portion 244 and proximal portion 246 respectively.

The distal portion 244 may comprise the laterally outermost surface 242 of the sealing element 240. Accordingly, the maximum lateral dimension D₂₄₄ of the distal portion 244 may be a maximum lateral dimension of the sealing element 240.

The proximal portion 246 may comprise at least one laterally-extending projection 241. The laterally-extending projection(s) 241 may extend at least partially or entirely about a periphery of the proximal portion 246.

The sealing element 240 may be formed of a polymeric material such as the polymeric materials previously described with reference to sealing element 140.

When the stopper 130 and the sealing element 240 are assembled together to form the stopper assembly 220, a portion of the sealing element 240 is disposed within the cavity 138 of the stopper 130. In particular, the proximal portion 246 of the sealing element 240 may be disposed in the cavity 138 of the stopper 130.

When the proximal portion 246 of the sealing element 240 is disposed in the cavity 138 of the stopper 130, the laterally-extending projection(s) 246 are disposed in the laterally-extending groove(s) 131. Disposing the laterally-extending projection(s) 246 of the sealing element 240 in the laterally-extending groove(s) 131 of the stopper 130 provides a coupling mechanism between the sealing element 240 and the stopper 130. Thus, when the stopper assembly 220 is axially displaced within the barrel 202 in operation, the sealing element 240 and the stopper 130 remain coupled together.

When the stopper assembly 220 is disposed in the barrel 202, the laterally outer surface 132 of the stopper 130 is directly engaged with the inner surface 108 of the barrel 102.

The lateral dimension D130 of the stopper 130 may be greater than the maximum lateral dimension D208 and the minimum lateral dimension D203, 205. Due to the dimensions of the stopper 130 relative to the dimensions of the barrel 202, the stopper 130 is laterally compressed within the barrel 202 when the stopper assembly 220 is disposed therein. The lateral compression of the stopper 130 exerts a compressive force on the proximal portion 246 of the sealing element 240 disposed in the cavity 138 of the stopper 130. In addition to or as an alternative to providing the laterally-extending grooves 131 and projections 241, the laterally compressive force of the stopper 130 on the sealing element 240 provides a coupling mechanism between the sealing element 240 and the stopper 130. Thus, when the stopper assembly 220 is displaced within the barrel 202, the sealing element 240 and the stopper 130 remain coupled together.

When the stopper assembly 220 is disposed in the barrel 202, the sealing element 240 is located axially distal relative to the stopper 130. More particularly, the distal portion 244 of the sealing element 240 may abut against the distal surface 134 of the stopper 130. By disposing the sealing element 240 axially distal relative to the stopper 130, the sealing element 240 is in contact with the medicinal fluid also disposed within the barrel 202. Thus, the stopper 130 is sealingly isolated from the medicinal fluid by the sealing element 240.

In some embodiments, when the stopper assembly 220 is disposed in the barrel 202, the laterally outermost surface 242 of the sealing element 240 may be disengaged from the inner surface 208 of the barrel 202. The maximum lateral dimension D₂₀₈ of the inner surface 208 of the barrel 202 may be greater than the maximum lateral dimension D₂₄₆ of the proximal portion 246 of the sealing element 240. By dimensioning the maximum lateral dimension D₂₄₆ of the proximal portion 246 of the sealing element 240 to be less than the lateral dimension D₂₀₈ of the inner surface 208 of the barrel 202, the surface roughness of the laterally outermost surface 242 is irrelevant to the predetermined actuating force that must be applied to the plunger rod 110 to axially displace the stopper assembly 220.

In other embodiments, when the stopper assembly 220 is disposed in the barrel 202, the laterally outermost surface 242 of the sealing element 240 may be directly engaged from the inner surface 208 of the barrel 202. Thus, the maximum lateral dimension D₂₄₆ of the proximal portion 246 may be selected as previously described with reference to the sealing element 140.

In either embodiment, the sealing element 240 may abut against the retaining ring 205 in the storage position such that the sealing element 240 is located proximal relative to the retaining ring 205 and distal relative to the retaining ring 203.

The injection device 200 may further comprise the plunger rod 110, as previously described with reference to FIG. 1A. The distal end 112 of the plunger rod 110 may be disposed in the barrel 202. The distal end 112 of the plunger rod 110 may be coupled to the stopper assembly 220. Accordingly, the stopper 130 may be disposed between the flange 113 of the plunger rod 110 and the sealing element 240.

When the stopper assembly 220 is disposed in the storage position in the barrel 202, each of the sealing element 240, the stopper 130, and the flange 113 of the plunger 110 may be located axially between the retaining rings 203, 205. The sealing element 240 may abut against the another retaining ring 205.

Further, the axial distance D₂₂₀ may be selected such that the flange 113 of the plunger rod 110 and the distal portion 244 of the sealing element 240 apply an axially compressive force on the stopper 130. The stopper 130 may apply an opposing axial force on the flange 113 of the plunger rod 110 and the distal portion 244 of the sealing element 240.

This opposing force applied by the stopper 130 forms a fluid tight interface between the sealing element 240 and the another retaining ring 205 of the barrel 202. Thus, in addition to the axial location of the sealing element 240 relative to the stopper 130, the dimension of the sealing element 240 relative to the dimension of the another retaining ring 205 and the force of the stopper 130 sealingly isolates the stopper 130 from the medicinal fluid. In other words, the medicinal fluid disposed in the barrel 202 is in contact with the sealing element 240 and cannot contact the stopper 130 due to the fluid tight interface between the sealing element 240 and the another retaining ring 205 when the stopper assembly 220 is in the storage position. As a result of the isolation of the stopper 130 from the medicinal fluid, the potency of the medicinal fluid remains unchanged when the medicinal fluid is stored within the barrel 102 over time.

As previously described herein, the maximum lateral dimension D₂₄₄ of the sealing element 240 and the lateral dimension D₁₃₀ of the stopper 130 are greater than the minimum lateral dimension D₂₀₅ of the retaining ring 103. The sealing element 240 and the stopper 130 may be compressed to allow passage of the stopper assembly 220 between and distally beyond the retaining ring 205.

In operation, typically during injection, the stopper assembly 220 is axially displaced from the storage position to the use position. FIG. 4A illustrates the stopper assembly 220 in the storage position. As previously described, the stopper assembly 220 is located axially proximal relative to the retaining ring 205 in the storage position. To axially displace the stopper assembly 220 and to administer the medicinal fluid to a patient, a force is applied by a user on the proximal end 114 of the plunger rod 110. The applied force must be greater than or equal to a predetermined actuating force. One or more of the maximum lateral dimension D₂₄₄ of the sealing element 240, the lateral dimension D₁₃₀ of the stopper 130, and the minimum lateral dimension D₂₀₅ of the retaining ring may be selected to obtain a desired, predetermined actuating force.

While the stopper assembly 220 is axially displaced within the barrel 202 (e.g., in a use position), the stopper 130 may remain sealingly isolated from the medicinal fluid or may contact some medicinal fluid depending on the maximum lateral dimension D₂₄₄ of the sealing element 240 as previously described herein. Even if the medicinal fluid contacts the stopper 130 in the use position, there is insufficient time during the operation of the injection device 200 for the medicinal fluid and the stopper 130 to affect the potency of the drug.

The plunger rod 110 may continue to be axially displaced until the sealing element 240 abuts against a distal end of the inner surface 208 of the barrel 202. For injection devices lacking the sealing element 240, a syringe-induced blood reflux may occur. The presence of the sealing element 240 and the material composition of the sealing element 240 prevents such compression and subsequent rebound of the stopper 130 because the stopper 130 does not abut against the distal end of the inner surface 108 of the barrel 102. Thus, a further advantage of the stopper assembly 220 is the avoidance of blood reflux.

While the present disclosure has been described herein with respect to certain illustrated embodiments, those of ordinary skill in the art will recognize and appreciate that it is not so limited. Rather, many additions, deletions, and modifications to the illustrated embodiments may be made without departing from the scope of the disclosure, including legal equivalents thereof. In addition, features from one embodiment may be combined with features of another embodiment while still being encompassed within the scope of the invention as defined in the appended claims.

## Claims

1. An injection device (200) comprising:
a barrel (202) extending axially between a distal end (204) and a proximal end (206), the barrel (202) configured to contain a medicinal fluid, the barrel (202) comprising a first retaining ring (203) proximate to the proximal end (206);
a plunger rod (110) having a distal end (112) and a proximal end (114), the distal end (112) of the plunger rod (110) disposed in the barrel (202); and
a stopper assembly (220) disposed in the barrel (202), the stopper assembly comprising:
a stopper (130) having a laterally outermost surface (132) configured to be directly engaged with an inner surface (208) of the barrel (202); and
a sealing element (240) coupled to and axially distal relative to the stopper (130) in the barrel (202), the sealing element (240) having a laterally outermost surface (242) configured to be directly engaged with the inner surface (208) of the barrel (202) and to sealingly isolate the stopper (130) from the medicinal fluid in the barrel (202)
the injection device **characterized in that**:
the barrel (202) comprises a second retaining ring (205) located proximate to the proximal end (206) of the barrel (202), the first retaining ring (203) axially spaced apart from and located proximal relative to the second retaining ring (205), the first and second retaining rings (203, 205) defining a minimum lateral dimension (D₂₀₃, D₂₀₅) of the inner surface (208) of the barrel (202); and
wherein each of the sealing element (240), the stopper (130), and a flange (113) of the plunger rod (110) is located between the first and second retaining rings (203, 205) in the storage position such that the stopper (130) is axially compressed between the sealing element (240) and the flange (113) of the plunger (110).

2. The injection device (200) of claim 1, wherein the sealing element (240) comprises a polymeric material chemically inert to the medicinal fluid to be disposed within the barrel (202).

3. The injection device (200) of either of claims 1 or 2, wherein the sealing element (240) comprises a distal portion (244) and a proximal portion (246), the distal portion (244) having a maximum lateral dimension (D₂₄₄) greater than a maximum lateral dimension (D₂₄₆) of the proximal portion (246).

4. The injection device (200) of any one of claims 1-3, wherein:
the stopper (130) has a distal surface (134) and a proximal surface (136),
the stopper (130) comprises a cavity (138) extending into the stopper (130) from the distal surface (134) thereof, and
the proximal portion (246) of the sealing element (240) is disposed in the cavity (138) of the stopper (130).

5. The injection device (200) of claim 4, wherein the cavity (138) comprises at least one laterally-extending groove (131) and the proximal portion (246) of the sealing element (240) comprises at least one laterally-extending projection (241), the at least one laterally-extending projections (241) received in and engaged with the laterally-extending grooves (131) to couple the stopper (130) and the sealing element (240).

6. The injection device (200) of any one of claims 1-5, wherein the distal portion (244) of the sealing element (240) is hemispherical in shape, conical in shape, or disc-shaped.

7. The injection device (200) of any one of claims 1-7, wherein the stopper (130) is sealingly enclosed between the sealing element (240) and the plunger (110).

8. The injection device (200) of any one of claims 1-7, wherein a maximum lateral dimension (D₂₀₈) of the inner surface (208) of the barrel (202) is less than the maximum lateral dimension (D₂₄₄) of the distal portion (244) of the sealing element (240) such that the distal portion (244) of the sealing element (240) is laterally compressed within the barrel (202) in the storage position.

9. The injection device (200) of claim 8, wherein a maximum lateral dimension (D₁₃₀) of the stopper (130) is greater than the maximum lateral dimension (D₂₀₈) of the inner surface (208) of the barrel (202) such that the stopper (130) is laterally compressed within the barrel (202), and wherein the lateral compression of the stopper (130) applies a compressive retaining force on the proximal portion (246) of the sealing element (240) disposed in the cavity (138) of the stopper (130).

10. The injection device (200) of any of claims 1-9, wherein:
the stopper assembly (220) is configured to be axially movable from a storage position to a use position within the barrel (202).

11. The injection device (200) of claim 8, wherein the maximum lateral dimension (D₂₀₈) of the inner surface (208) of the barrel (202) axially distal relative to the first and second retaining rings (203, 205) is greater than the maximum lateral dimension (D₂₄₄) of the distal portion (244) of the sealing element (240) such that the laterally outermost surface (242) of the sealing element (240) is disengaged from the inner surface (208) of the barrel (202) in the use position.

## Patentansprüche

1. Injektionsvorrichtung (200), bestehend aus:
einem Zylinder (202), der sich axial zwischen einem distalen Ende (204) und einem proximalen Ende (206) erstreckt, wobei der Zylinder (202) so eingerichtet ist, dass er eine Arzneimittelflüssigkeit enthält, wobei der Zylinder (202) einen ersten Sicherungsring (203) unweit des proximalen Endes (206) umfasst;
einem Spritzenkolben (110) mit einem distalen Ende (112) und einem proximalen Ende (114), wobei das distale Ende (112)
des Spritzenkolbens (110) im Zylinder (202) angeordnet ist; und
einer Stopfenbaugruppe (220), die im Zylinder (202) angeordnet ist, wobei die Stopfenbaugruppe Folgendes umfasst:
einen Stopfen (130) mit einer seitlichen Außenfläche (132), die so angeordnet ist, dass sie direkt mit einer Innenfläche (208) des Zylinders (202) in Berührung kommt; und
ein Dichtungselement (240), verbunden mit und axial relativ zu dem Stopfen (130) im Zylinder (202), wobei das Dichtungselement (240) eine seitliche Außenfläche (242) aufweist, die so angeordnet ist, dass sie direkt mit der Innenfläche (208) des Zylinders (202) in Berührung kommt und den Stopfen (130) dicht von der Arzneimittelflüssigkeit im Zylinder (202) isoliert
die Einspritzvorrichtung, **dadurch gekennzeichnet, dass**:
der Zylinder (202) einen zweiten Sicherungsring (205) umfasst, der sich unweit des proximalen Endes (206) des Zylinders (202) befindet, wobei sich der erste Sicherungsring (203) axial vom zweiten Sicherungsring (205) entfernt und proximal relativ zu diesem befindet, wobei der erste und der zweite Sicherungsring (203, 205) eine seitliche Mindestausdehnung (D₂₀₃, D₂₀₅) der Innenfläche (208) des Zylinders (202) definieren; und
wobei sich in der Lagerposition das Dichtungselement (240), der Stopfen (130) und ein Flansch (113) des Spritzenkolbens (110) jeweils zwischen dem ersten und zweiten Sicherungsring (203, 205) befinden, sodass der Stopfen (130) axial zwischen dem Dichtungselement (240) und dem Flansch (113) des Kolbens (110) komprimiert ist.

2. Injektionsvorrichtung (200) nach Anspruch 1, wobei das Dichtungselement (240) ein Polymermaterial umfasst, das chemisch inert gegenüber der Arzneimittelflüssigkeit innerhalb des Zylinders (202) ist.

3. Injektionsvorrichtung (200) nach einem der Ansprüche 1 oder 2, wobei das Dichtungselement (240) aus einem distalen Abschnitt (244) und einem proximalen Abschnitt (246) besteht und der distale Abschnitt (244) eine seitliche Maximalausdehnung (D₂₄₄) aufweist, die größer ist als die seitliche Maximalausdehnung (D₂₄₆) des proximalen Abschnitts (246).

4. Injektionsvorrichtung (200) nach einem der Ansprüche 1 bis 3, wobei:
der Stopfen (130) eine distale Oberfläche (134) und eine proximale Oberfläche (136) besitzt,
der Stopfen (130) einen Hohlraum (138) aufweist, der sich von der distalen Oberfläche (134) aus in den Stopfen (130) hinein erstreckt, und
der proximale Abschnitt (246) des Dichtungselements (240) im Hohlraum (138) des Stopfens (130) angeordnet ist.

5. Injektionsvorrichtung (200) nach Anspruch 4, wobei der Hohlraum (138) mindestens eine seitlich verlaufende Nut (131) und der proximale Abschnitt (246) des Dichtungselements (240) mindestens einen seitlich verlaufenden Vorsprung (241) umfasst, wobei der mindestens eine seitlich verlaufende Vorsprung (241) von der seitlich verlaufenden Nut (131) aufgenommen und erfasst wird, um den Stopfen (130) und das Dichtungselement (240) zu verbinden.

6. Injektionsvorrichtung (200) nach einem der Ansprüche 1 bis 5, wobei der distale Abschnitt (244) des Dichtungselements (240) halbkugelförmig, konisch oder scheibenförmig ist.

7. Injektionsvorrichtung (200) nach einem der Ansprüche 1 bis 6, wobei der Stopfen (130)
dicht zwischen dem Dichtungselement (240) und dem Kolben (110) eingeschlossen ist.

8. Injektionsvorrichtung (200) nach einem der Ansprüche 1 bis 7, wobei die seitliche Maximalausdehnung (D₂₀₈) der Innenfläche (208) des Zylinders (202) kleiner ist als die seitliche Maximalausdehnung (D₂₄₄) des distalen Abschnitts (244) des Dichtungselements (240), sodass in der Lagerposition der distale Abschnitt (244) des Dichtungselements (240) seitlich innerhalb des Zylinders (202) komprimiert ist.

9. Injektionsvorrichtung (200) nach Anspruch 8, wobei ein die seitliche Maximalausdehnung (D₁₃₀) des Stopfens (130) größer ist als die seitliche Maximalausdehnung (D₂₀₈) der Innenfläche (208) des Zylinders (202), sodass der Stopfen (130) seitlich innerhalb des Zylinders (202) komprimiert ist, und wobei die seitliche Kompression des Stopfens (130) eine komprimierende Haltekraft auf den proximalen Abschnitt (246) des im Hohlraum (138) des Stopfens (130) befindlichen Dichtungselements (240) ausübt.

10. Injektionsvorrichtung (200) nach einem der Ansprüche 1 bis 9, wobei:
die Stopfenbaugruppe (220) so angeordnet ist, dass sie innerhalb des Zylinders (202) axial von einer Lagerposition zu einer Gebrauchsposition beweglich ist.

11. Einspritzvorrichtung (200) nach Anspruch 8, wobei die seitliche Maximalausdehnung (D₂₀₈) der Innenfläche (208) des Zylinders (202) axial distal zum ersten und zweiten Sicherungsring (203, 205) größer ist als die seitliche Maximalausdehnung (D₂₄₄) des distalen Abschnitts (244) des Dichtungselements (240), sodass die seitliche Außernfläche (242) des Dichtungselements (240) in der Gebrauchsstellung von der Innenfläche (208) des Zylinders (202) abgelöst ist.

## Revendications

1. Dispositif d'injection (200) comprenant :
un cylindre (202) s'étendant axialement entre une extrémité distale (204) et une extrémité proximale (206), le cylindre (202) configuré pour contenir un fluide médicinal, le cylindre (202) comprenant un premier anneau de retenue (203) à proximité de l'extrémité proximale (206) ;
une tige de piston (110) ayant une extrémité distale (112) et une extrémité proximale (114), l'extrémité distale (112) de la tige de piston (110) étant disposée dans le cylindre (202) ; et
un ensemble de bouchon (220) disposé dans le cylindre (202), l'ensemble de bouchon comprenant :
un bouchon (130) ayant une surface extérieure latérale (132) configurée pour être directement engagée avec une surface intérieure (208) du cylindre (202) ; et
un élément d'étanchéité (240) couplé à et axialement distal par rapport au bouchon (130) dans le cylindre (202), l'élément d'étanchéité (240) ayant une surface extérieure latérale (242) configurée pour être directement engagée avec la surface intérieure (208) du cylindre (202) et pour isoler de manière étanche le bouchon (130) du fluide médicinal dans le cylindre (202) ;
le dispositif d'injection étant **caractérisé en ce que** :
le cylindre (202) comprend un second anneau de retenue (205) situé à proximité de l'extrémité proximale (206) du cylindre (202), le premier anneau de retenue (203) axialement espacé du second anneau de retenue (205) et situé à proximité de celui-ci, les premier et second anneaux de retenue (203, 205) définissant une dimension latérale minimale (D₂₀₃, D₂₀₅) de la surface intérieure (208) du cylindre (202) ; et
dans lequel chacun de l'élément d'étanchéité (240), du bouchon (130) et d'une bride (113) de la tige de piston (110) est situé entre les premier et second anneaux de retenue (203, 205) dans la position de stockage, de sorte que le bouchon (130) est comprimé axialement entre l'élément d'étanchéité (240) et la bride (113) du piston (110).

2. Le dispositif d'injection (200) de la revendication 1, dans lequel l'élément d'étanchéité (240) comprend un matériau polymère chimiquement inerte vis-à-vis du fluide médicinal à disposer dans le cylindre (202).

3. Le dispositif d'injection (200) de l'une des revendications 1 ou 2, dans lequel l'élément d'étanchéité (240) comprend une partie distale (244) et une partie proximale (246), la partie distale (244) ayant une dimension latérale maximale (D₂₄₄) supérieure à une dimension latérale maximale (D₂₄₆) de la partie proximale (246).

4. Le dispositif d'injection (200) de l'une des revendications 1 à 3, dans lequel :
le bouchon (130) a une surface distale (134) et une surface proximale (136),
le bouchon (130) comprend une cavité (138) qui s'étend à l'intérieur du bouchon (130) à partir de sa surface distale (134), et
la partie proximale (246) de l'élément d'étanchéité (240) est disposée dans la cavité (138) du bouchon (130).

5. Le dispositif d'injection (200) de la revendication 4, dans lequel la cavité (138) comprend au moins une rainure (131) d'extension latérale et la partie proximale (246) de l'élément d'étanchéité (240) comprend au moins une saillie (241) d'extension latérale, les au moins une saillie d'extension latérale (241) étant reçues dans les rainures d'extension latérale (131) et engagées dans celles-ci pour coupler le bouchon (130) et l'élément d'étanchéité (240).

6. Le dispositif d'injection (200) de l'une des revendications 1 à 5, dans lequel la partie distale (244) de l'élément d'étanchéité (240) est de forme hémisphérique, conique ou en forme de disque.

7. Le dispositif d'injection (200) de l'une des revendications 1 à 7, dans lequel le bouchon (130) est enfermé de manière étanche entre l'élément d'étanchéité (240) et le piston (110).

8. Le dispositif d'injection (200) de l'une des revendications 1 à 7, dans lequel une dimension latérale maximale (D₂₀₈) de la surface intérieure (208) du cylindre (202) est inférieure à la dimension latérale maximale (D₂₄₄) de la partie distale (244) de l'élément d'étanchéité (240) de sorte que la partie distale (244) de l'élément d'étanchéité (240) est comprimée latéralement à l'intérieur du cylindre (202) dans la position de stockage.

9. Le dispositif d'injection (200) de la revendication 8, dans lequel une dimension latérale maximale (D₁₃₀) du bouchon (130) est supérieure à la dimension latérale maximale (D₂₀₈) de la surface intérieure (208) du cylindre (202) de sorte que le bouchon (130) est comprimé latéralement à l'intérieur du cylindre (202), et dans lequel la compression latérale du bouchon (130) applique une force de retenue compressive sur la partie proximale (246) de l'élément d'étanchéité (240) disposé dans la cavité (138) du bouchon (130).

10. Le dispositif d'injection (200) de l'une des revendications 1 à 9, dans lequel :
l'ensemble de bouchon (220) est configuré pour être déplacé axialement d'une position de stockage à une position d'utilisation à l'intérieur du cylindre (202).

11. Le dispositif d'injection (200) de la revendication 8, dans lequel la dimension latérale maximale (D₂₀₈) de la surface intérieure (208) du cylindre (202) axialement distale par rapport aux premier et deuxième anneaux de retenue (203, 205) est supérieure à la dimension latérale maximale (D₂₄₄) de la partie distale (244) de l'élément d'étanchéité (240) de sorte que la surface latéralement la plus extérieure (242) de l'élément d'étanchéité (240) est désengagée de la surface intérieure (208) du cylindre (202) dans la position d'utilisation.
